# EUROPEAN PATENT APPLICATION

(11) **EP 4 321 873 A1**
(43) Date of publication of application: **14.02.2024**
(21) Application number: 22784087.3
(22) Date of filing: 07.04.2022
(51) Int. Cl.: G01N 33/76, G01N 33/48, G01N 33/50, G01N 21/78

(54) **EARLY PREGNANCY TESTING PEN CASING AND EARLY PREGNANCY TESTING PEN**

(30) Priority: 08.04.2021 CN 202120713738 U
(71) Applicant: Abbott Diagnostics (Shanghai) Co., Ltd., Shanghai 201203 (CN)
(72) Inventor: DU, Demin, Shanghai 201203 (CN)
(74) Representative: Redhouse, Juliet Lauren
(86) International application number: PCT/CN2022/085504
(87) International publication number: WO 2022/214021

(57) **Abstract**

Provided are an early pregnancy testing pen casing and an early pregnancy testing pen. The early pregnancy testing pen includes an early pregnancy testing pen casing, a test strip (3), and a water absorption piece (4). The early pregnancy testing pen casing includes a first casing body (1) and a second casing body (2). The second casing body (2) is divided into a functional section (21) and a handheld section (22) arranged in a length direction of the second casing body (2). The first casing body (1) is opposite to the functional section (21) so that an accommodating space for accommodating the test strip (3) is formed between the first casing body (1) and the functional section (21). The accommodating space has an opening for the water absorption piece (4) located in the accommodating space to extend out of the early pregnancy testing pen casing through the opening. The handheld section (22) is used for being held by a user, and the handheld section (22) does not overlap the first casing body (1). The present application reduces the material consumption.

## Description

### CROSS-REFERENCE TO RELATED APPLICATIONS

The present application claims the priority of Chinese Patent Application No. 202120713738.8, filed on April 8, 2021 and entitled "EARLY PREGNANCY TESTING PEN CASING AND EARLY PREGNANCY TESTING PEN", which is incorporated herein by reference.

### Technical Field

The present application belongs to the technical field of early pregnancy testing, and particularly relates to an early pregnancy testing pen casing and an early pregnancy testing pen.

### Background Art

An early pregnancy testing pen is used for diagnosing early pregnancy for women. The early pregnancy testing pen is usually constructed of a plastic casing wrapped around a test strip. Since the early pregnancy testing pen is a disposable item, the disposal of the plastic casing will cause environmental pollution.

### Summary of the Invention

An objective of the present application is to provide an early pregnancy testing pen casing and an early pregnancy testing pen to overcome the defects in the prior art.

In order to solve the above technical problem, the present application adopts the following technical solution: an early pregnancy testing pen casing includes a first casing body and a second casing body. The second casing body is divided into a functional section and a handheld section arranged in a length direction of the second casing body. The first casing body is opposite to the functional section so that an accommodating space for accommodating a test strip is formed between the first casing body and the functional section. The accommodating space has an opening for a water absorption piece located in the accommodating space to extend out of the early pregnancy testing pen casing through the opening. The handheld section is used for being held by a user. The handheld section does not overlap the first casing body.

In some embodiments, the second casing body has a casing plate, and a plate surface of the casing plate located at the handheld section is provided with at least one through hole running through up and down.

In some embodiments, there are a plurality of through holes that are arranged in the length direction of the second casing body.

In some embodiments, the through hole extends in a width direction of the second casing body.

In some embodiments, reinforcing ribs in one-to-one correspondence to the through holes are further arranged in the handheld section. The reinforcing ribs are arranged on the casing plate. The reinforcing ribs are each arranged outside a boundary of the corresponding through hole away from the functional section, or outside a boundary of the corresponding through hole facing the functional section. The reinforcing ribs extend in the width direction of the second casing body, and the reinforcing ribs and the first casing body are located on a same side of the plate surface of the casing plate.

In some embodiments, the first casing body and the second casing body are each an integrally formed plastic piece.

In some embodiments, the first casing body is a lower casing, and the second casing body is an upper casing; or the first casing body is an upper casing, and the second casing body is a lower casing.

In some embodiments, the first casing body and the second casing body are fixed together through at least locating holes and locating pillars in pairs.

In order to solve the above technical problem, the present application adopts the following technical solution: an early pregnancy testing pen includes the test strip, the water absorption piece and the early pregnancy testing pen casing described above. A part of the water absorption piece is located in the accommodating space, and the water absorption piece extends out of the early pregnancy testing pen casing through an opening of the accommodating space. The water absorption piece is connected to the test strip.

Compared with the prior art, the present application has the following beneficial effects: since the first casing body does not need to cover the whole of the second casing body, the overall material consumption of the first casing body and the second casing body is reduced, thereby reducing the material cost and the environmental pollution.

### Brief Description of the Drawings

FIG. 1 is a schematic structural diagram of a first casing body of an early pregnancy testing pen casing according to an embodiment of the present application;
FIG. 2 and FIG. 3 are respectively schematic structural diagram s of a second casing body of the early pregnancy testing pen casing from different viewing angles according to an embodiment of the present application; and
FIG. 4 is a schematic diagram showing an internal structure of the early pregnancy testing pen according to an embodiment of the present application.

In the figures: 1, first casing body; a, locating pillar; b, locating hole; 2, second casing body; 21, functional section; 22, handheld section; 220, casing plate; 221, through hole; 222, reinforcing rib; 3, test strip; 4, water absorption piece.

### Detailed Description of the Invention

In the present application, it should be understood that terms such as "include" or "have" are intended to indicate the existence of the disclosed features, numbers, steps, actions, components, parts or combinations thereof in this specification, but do not exclude the possibility of the existence of one or more other features, numbers, steps, actions, components, parts or combinations thereof.

In addition, it should also be noted that the embodiments in the present application and the features in the embodiments may be combined with each other in the case of no conflict. The present application will be described in detail below with reference to the accompanying drawings and in conjunction with the embodiments.

The present application will be further described below with reference to embodiments shown in the accompanying drawings.

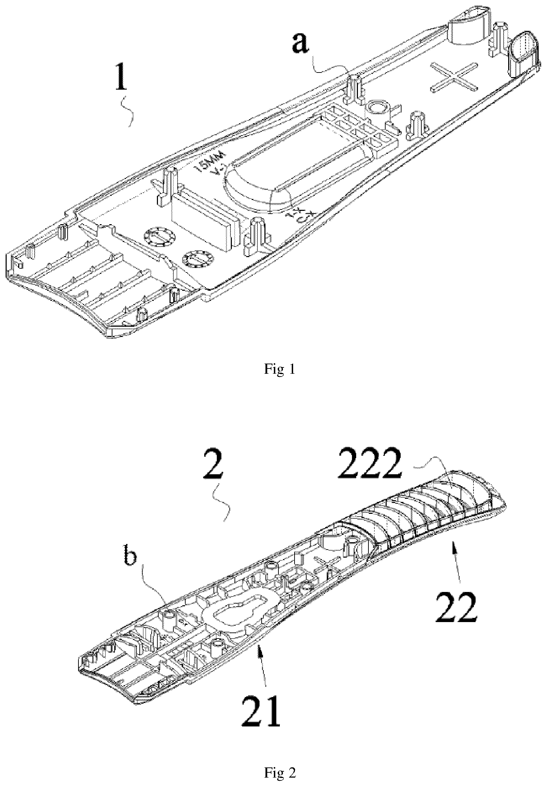

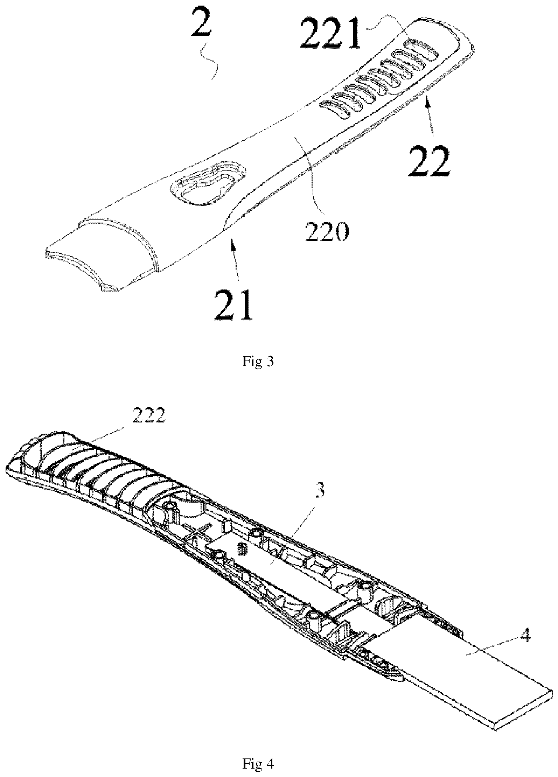

Referring to FIG. 1 to FIG. 3 in conjunction with FIG. 4, an early pregnancy testing pen casing of the present application includes a first casing body 1 and a second casing body 2. The second casing body 2 is divided into a functional section 21 and a handheld section 22 arranged in a length direction of the second casing body 2. The first casing body 1 is opposite to the functional section 21 so that an accommodating space for accommodating a test strip 3 is formed between the first casing body 1 and the functional section 21. The accommodating space has an opening for a water absorption piece 4 located in the accommodating space to extend out of the early pregnancy testing pen casing through the opening. The handheld section 22 is used for being held by a user. The handheld section 22 does not overlap the first casing body 1.

The first casing body 1 has a shorter length, and is buckled on the functional section 21 of the second casing body 2. The second casing body 2 has an elongated appearance.

"The handheld section 22 does not overlap the first casing body 1" means that when the early pregnancy testing pen casing is laid flat on a horizontal desktop, orthographic projections of the handheld section and the first casing body in the horizontal plane do not overlap.

A test strip 3, a desiccant (not shown) and the like that constitute a conventional structure of an early pregnancy testing pen may be arranged in the accommodating space formed by the first casing body 1 and the second casing body 2. When the early pregnancy testing pen casing is used to assemble an early pregnancy testing pen, a part of the water absorption piece 4 is located in the accommodating space so as to be connected to the test strip 3, and another part extends out from an end of the functional section 21 facing away from the handheld section 22. The part that extends out is used for receiving urine, so that the urine is transferred to the test strip 3 via the water absorption piece 4.

When the early pregnancy testing pen is in use, the user holds the handheld section 22, and then the exposed water absorption piece 4 receives the urine. The water absorption piece 4 transfers the urine to the test strip 3 in the accommodating space.

Since the first casing body 1 does not need to cover the whole of the second casing body 2, the overall material consumption of the first casing body 1 and the second casing body 2 is reduced, thereby reducing the material cost and the environmental pollution.

In some embodiments, referring to FIG. 3, the second casing body 2 has a casing plate 220, and a plate surface of the casing plate 220 located at the handheld section 22 is provided with at least one through hole 221 running through up and down.

The "up and down" direction here refers to the up-and-down direction when the early pregnancy testing pen casing is flatly on the horizontal plane.

This further reduces the volume of the second casing body 2, thereby reducing the material consumption. There may be one or a plurality of through holes 221. A shape of the through holes 221 may also be designed flexibly.

For example, in some embodiments, referring to FIG. 3, there are a plurality of through holes 221 that are arranged in the length direction of the second casing body 2. That is, the through holes 221 are arranged in a row, which not only achieves the aesthetic effect, but also further reduces the material consumption of the second casing body 2.

In some embodiments, referring to FIG. 3, the through hole 221 extends in a width direction of the second casing body 2. That is, the through hole 221 is elongated. Of course, the through hole 221 may also be circular or square or in other shapes. The elongated through hole can achieve the effect of beautiful appearance.

In some embodiments, referring to FIG. 2, reinforcing ribs 222 in one-to-one correspondence to the through holes 221 are further arranged in the handheld section 22 of the second casing body 2. The reinforcing ribs 222 are arranged on the casing plate 220. The reinforcing ribs 222 are each arranged outside a boundary of the corresponding through hole 221 away from the functional section 21, or outside a boundary of the corresponding through hole 221 facing the functional section 21. The reinforcing ribs 222 extend in the width direction of the second casing body 2, and the reinforcing ribs 222 and the first casing body 1 are located on a same side of the plate surface of the casing plate 220.

That is, along the direction from the functional section 21 to the handheld section 22 in the second casing body 2, the through hole 221, the reinforcing rib 222, the through hole 221, the reinforcing rib 222, ..., or the reinforcing rib 222, the through hole 221, the reinforcing rib 222, the through hole 221, ..., are sequentially arranged.

With this arrangement, the second casing body 2 can have a neat and beautiful structure, and the overall thickness of the handheld section 22 is increased, which is convenient for the user to grasp.

In some embodiments, the first casing body 1 and the second casing body 2 are each an integrally formed plastic piece. Thus, the first casing body 1 and the second casing body 2 can be made according to the existing process.

In some embodiments, the first casing body 1 is a lower casing, and the second casing body 2 is an upper casing. That is, when in use, the second casing body 2 faces upward, and the first casing body 1 faces downward, which makes the handheld section more convenient for the user to grasp. Alternatively, the first casing body 1 is an upper casing, and the second casing body 2 is a lower casing.

In some embodiments, the first casing body 1 and the second casing body 2 are fixed together through at least locating holes b and locating pillars a in pairs. The locating holes b may be arranged in either the first casing body 1 or the second casing body 2, or in both the first casing body 1 and the second casing body 2. The present application does not limit how the first casing body 1 and the second casing body 2 are assembled together, which can be designed according to the prior art.

Referring to FIG. 4 in conjunction with FIG. 1 to FIG. 3, an embodiment of the present application further provides an early pregnancy testing pen, including the test strip 3, the water absorption piece 4 and the early pregnancy testing pen casing described above. The test strip 3 is arranged in the accommodating space. A part of the water absorption piece 4 is located in the accommodating space, and the water absorption piece extends out of the early pregnancy testing pen casing through the opening of the accommodating space. The water absorption piece 4 is connected to the test strip 3.

The first casing body 1 is not shown in the early pregnancy testing pen shown in FIG. 4. The first casing body 1 shown in FIG. 1 and the structure shown in FIG. 4 can be buckled together to complete the assembly of the early pregnancy testing pen.

All embodiments in the present application are described in a progressive manner. For the same or similar parts between the embodiments, reference may be made to each other. Each embodiment focuses on differences from other embodiments.

The scope of protection of the present application is not limited to the above-mentioned embodiments. Obviously, those skilled in the art can make various modifications and variations to the present application without departing from the spirit and scope of the present application. If such modifications and variations fall within the scope of the claims of the present application and the equivalent technologies thereof, the present application is also intended to cover such modifications and variations.

## Claims

1. An early pregnancy testing pen casing, comprising a first casing body (1) and a second casing body (2), wherein the second casing body (2) is divided into a functional section (21) and a handheld section (22) arranged in a length direction of the second casing body (2); the first casing body (1) is opposite to the functional section (21) so that an accommodating space for accommodating a test strip (3) is formed between the first casing body (1) and the functional section (21); the accommodating space has an opening for a water absorption piece (4) located in the accommodating space to extend out of the early pregnancy testing pen casing through the opening; the handheld section (22) is used for being held by a user; and the handheld section (22) does not overlap the first casing body (1).

2. The early pregnancy testing pen casing according to claim 1, wherein the second casing body (2) has a casing plate (220), and a plate surface of the casing plate (220) located at the handheld section (22) is provided with at least one through hole (221) running through up and down.

3. The early pregnancy testing pen casing according to claim 2, wherein there are a plurality of through holes (221) that are arranged in the length direction of the second casing body (2).

4. The early pregnancy testing pen casing according to claim 2 or 3, wherein the through hole (221) extends in a width direction of the second casing body (2).

5. The early pregnancy testing pen casing according to any one of claims 2 to 4, wherein reinforcing ribs (222) in one-to-one correspondence to the through holes (221) are further arranged in the handheld section (22), the reinforcing ribs (222) are arranged on the casing plate (220); the reinforcing ribs (222) are each arranged outside a boundary of the corresponding through hole (221) away from the functional section (21), or outside a boundary of the corresponding through hole (221) facing the functional section (21); and the reinforcing ribs (222) extend in the width direction of the second casing body (2), and the reinforcing ribs (222) and the first casing body (1) are located on a same side of the plate surface of the casing plate (220).

6. The early pregnancy testing pen casing according to any one of claims 1 to 5, wherein the first casing body (1) and the second casing body (2) are each an integrally formed plastic piece.

7. The early pregnancy testing pen casing according to any one of claims 1 to 6, wherein the first casing body (1) is a lower casing, and the second casing body (2) is an upper casing; or the first casing body (1) is an upper casing, and the second casing body (2) is a lower casing.

8. The early pregnancy testing pen casing according to any one of claims 1 to 7, wherein the first casing body (1) and the second casing body (2) are fixed together through at least locating holes (b) and locating pillars (a) in pairs.

9. An early pregnancy testing pen, including the test strip (3), the water absorption piece (4) and the early pregnancy testing pen casing according to any one of claims 1 to 8, wherein the test strip (3) is arranged in the accommodating space; a part of the water absorption piece (4) is located in the accommodating space, and the water absorption piece (4) extends out of the early pregnancy testing pen casing through an opening of the accommodating space; and the water absorption piece (4) is connected to the test strip (3).
